# EUROPEAN PATENT APPLICATION

(11) **EP 4 599 825 A1**
(43) Date of publication of application: **13.08.2025**
(21) Application number: 23910898.8
(22) Date of filing: 29.12.2023
(51) Int. Cl.: A61K 9/32, A61K 9/22, A61K 31/192, A61K 47/34, A61P 29/00

(54) **CONTROLLED RELEASE TABLET OF LOXOPROFEN SODIUM, PREPARATION METHOD, AND USE**

(30) Priority: 30.12.2022 CN 202211737747
(71) Applicant: Overseas Pharmaceuticals, Ltd., China 510530 (CN)
(72) Inventor: HUANG, Xiaofeng, Guangzhou, Guangdong 510530 (CN); WEN, Xiaoguang, Guangzhou, Guangdong 510530 (CN); ZHANG, Chenliang, Guangzhou, Guangdong 510530 (CN)
(74) Representative: Murgitroyd & Company
(86) International application number: PCT/CN2023/143062
(87) International publication number: WO 2024/140984

(57) **Abstract**

The present invention belongs to the technical field of pharmaceutical preparations, and in particular relates to a loxoprofen sodium controlled-release tablet, and a preparation method and use thereof. The controlled-release tablet includes a drug-containing immediate-release layer, a drug-containing tablet core layer and a drug-containing sustained-release layer. The drug-containing tablet core layer includes a plain tablet core, an enteric coating film and an isolation coating film. The preparation method includes: preparing a granule of a drug-containing immediate-release layer and a granule of drug-containing sustained-release layer respectively from a raw material of the drug-containing immediate-release layer and a raw material of the drug-containing sustained-release layer; subjecting a raw material of a plain tablet core to wet granulation, tableting, and then sequentially coating with a first layer of isolation coating , a second layer of enteric coating, and a third layer of isolation coating to obtain a drug-containing tablet core; and placing the drug-containing tablet core on the granule of the drug-containing sustained-release layer, pre-compressing, and then placing the granule of the drug-containing immediate-release layer on the pre-compressed tablet, tableting, and coating to obtain the tablet. In the present invention, the tablet structure, raw materials, coating and the like components of the core-coated tablet are designed to achieve the effect of sustained-release of the loxoprofen sodium tablet core, so that the tablet can be effective in vivo for 12 h and improve patient compliance.

## Description

### TECHNICAL FIELD

The present invention relates to the technical field of pharmaceutical preparations, and in particular relates to a loxoprofen sodium controlled-release tablet, and a preparation method and use thereof.

### BACKGROUND

Loxoprofen sodium tablets were first developed by Sankyo Co., Ltd., Japan and were marketed in Japan in July 1986 under the trade name "Loxonin^{®}" with a specification of 60 mg. It is the leading variety of non-steroidal anti-inflammatory drug in Japan. The drug was first marketed in China in 2003 under the trade name "LeSong^{®}". Compared with drugs of the same type in clinical, loxoprofen sodium has the advantages of faster onset of action, a stronger analgesic effect and wider application range in the treatment of painful diseases.

Studies have shown that the side effects of non-steroidal anti-inflammatory drugs, such as allergic responses, bleeding, and renal damage, are related to the exposure amount of a prototype drug or a metabolite thereof in plasma. Ordinary commercially-available preparations of loxoprofen sodium need to be taken 3 times a day, with poor medication compliance of patients; and at the same time, ordinary drug preparations are released too quickly in vivo, and the exposure amount of the prototype drug or a metabolite thereof in plasma is too large in a short period of time, and thus the blood drug concentration is unstable, which increases the side effects of the drug.

Chinese invention patent application CN105168167A discloses an osmotic pump controlled-release preparation containing loxoprofen sodium and a preparation method thereof, wherein the osmotic pump controlled-release preparation is an osmotic pump controlled-release tablet, including a tablet core and a semipermeable membrane with a drug release hole, which can reduce the number of dosing and improve patient compliance, but the onset speed, effectiveness and the like need to be further improved.

Chinese invention patent application CN102525989A discloses a loxoprofen sodium matrix sustained-release tablet with a regulating layer, which can release stably for 8 hours and has an ideal release effect. However, the number of daily dosing and the duration of effectiveness of it need to be further improved.

Chinese invention patent application CN105769773A discloses a loxoprofen sodium sustained-release micropellet, which consists of a drug-loaded pill core, an isolation layer wrapped on the drug-loaded pill core, and a sustained-release layer wrapped on the isolation layer. A weight ratio of the drug-loaded pill core, the isolation layer and the sustained-release layer is 80:1-15:2-20. The invention is a loxoprofen sodium sustained-release micropellet, the pH regulator concentration in an acidic isolation layer, the coating amount of the isolation layer and the coating amount of the sustained-release layer work together to achieve a slow in vitro release rate of the micropellet, and reduce the burst release phenomenon of loxoprofen sodium. The operation to achieve the sustained-release effect is complicated, and the micropellet cannot take effect quickly to achieve a rapid analgesic effect.

### SUMMARY

In view of the shortcomings of the prior art, the present invention provides a loxoprofen sodium controlled-release tablet and a preparation method thereof, which adopts a core-coated tablet design, optimizes the core-coated tablet structure, creatively designs and adjusts the coating formulation, and meanwhile optimizes the dosage distribution of immediate release and sustained release, combines the characteristics of immediate release and sustained release, so that after the patient takes the drug, the drug can take effect quickly and can also maintain its effectiveness for a long time by stably controlling the drug release. Thus, the blood drug concentration is kept stable.

An objective of the present invention is achieved through the following technical solutions.
a loxoprofen sodium controlled-release tablet is provided, which includes a drug-containing immediate-release layer, a drug-containing tablet core layer and a drug-containing sustained-release layer, wherein the drug-containing tablet core layer includes a plain tablet core, an enteric coating film and an isolation coating film; and the enteric coating film is not in direct contact with the tablet core, the drug-containing immediate-release layer and the drug-containing sustained-release layer.

Preferably, the enteric coating film is in direct contact only with the isolation coating film, and an exterior of the plain tablet core in the drug-containing tablet core layer is, from the inside to the outside, an isolation coating film 1, the enteric coating film and an isolation coating film 2.

Preferably, a raw material of the isolation coating film includes an isolation coating material selected from one or more of hydroxypropyl methylcellulose, hydroxypropyl cellulose, ethyl cellulose, and polyvinyl alcohol; the content of the isolation coating material in the isolation coating film is 5-20%, and a coating weight gain of the isolation coating film is 2%-10%.

Preferably, a raw material of the enteric coating film includes an enteric coating material selected from one or more of methacrylic acid, acrylate, methacrylate, polymethacrylic acid, polymethacrylate, trimethylaminoethyl methacrylate chloride, and polyvinyl acetate phthalate, the content of the enteric coating material in the enteric coating film is 5-35%, and the coating weight gain of the enteric coating film is 5%-30%; and preferably, the enteric coating material is a mixture of methacrylic acid and ethyl acrylate in a mass ratio of 1:1, and the coating weight gain is 15-25%; or the enteric coating material is a mixture of methacrylic acid and methyl methacrylate in a mass ratio of 1:2, and the coating weight gain is 6-30%.

Preferably, a raw material of the enteric coating film further includes an additive that includes a plasticizer and an anti-adherent agent in a mass ratio of 0.25-5: 1, preferably in a mass ratio of 0.25-4: 1; and the raw material of the isolation coating film further includes a plasticizer.
Preferably, the plasticizer is each selected from one or more of triethyl citrate, polyethylene glycol 6000, tributyl citrate and dibutyl sebacate;
preferably, the anti-adherent agent is selected from one or more of talc, magnesium stearate and glyceryl monostearate.
Preferably, a raw material of the plain tablet core includes the following components: loxoprofen sodium, a filler, a disintegrant and a lubricant, and preferably 20-65 parts of loxoprofen sodium, 30-70 parts of the filler, 1-5 parts of the disintegrant and 1-5 parts of the lubricant;
preferably, a raw material of the drug-containing immediate-release layer includes the following components: loxoprofen sodium, a filler, an adhesive, a disintegrant and a lubricant, and preferably 5-25 parts of loxoprofen sodium, 50-80 parts of the filler, 4-10 parts of the adhesive, 2-8 parts of the disintegrant, and 0.5-8 parts of the lubricant;
preferably, a raw material of the drug-containing sustained-release layer includes the following components: loxoprofen sodium, a sustained-release polymer, a filler, an adhesive and a lubricant, and preferably 10-35 parts of loxoprofen sodium, 5-60 parts of the sustained-release polymer, 25-40 parts of the filler, 5-15 parts of the adhesive, and 0.5-8 parts of the lubricant.
Preferably, the sustained-release polymer is selected from one or more of hydroxypropyl methylcellulose and a polysaccharide gum;
preferably, the hydroxypropyl methylcellulose is selected from one or more of E30LV, E50LV, K100LV, K4M, K15M and K100M;
preferably, the polysaccharide gum is selected from one or more of sodium alginate, arabic gum, xanthan gum and locust bean gum;
preferably, the sustained-release polymer accounts for 5-60%, preferably 7.5-40% of the sustained-release layer;
Preferably, the adhesive is each selected from one or more of sodium carboxymethylcellulose, povidone and hydroxypropyl methylcellulose; and preferably povidone.
Preferably, the disintegrant is selected from one or more of crosslinked sodium carboxymethyl starch, crosslinked sodium carboxymethyl cellulose, crosslinked polyvinylpyrrolidone (PVPP) and low-substituted hydroxypropyl cellulose (L-HPC); and preferably crosslinked sodium carboxymethyl starch or crosslinked sodium carboxymethyl cellulose.
Preferably, the filler is each selected from one or more of lactose, corn starch, pregelatinized starch and microcrystalline cellulose; and preferably lactose, pregelatinized starch or microcrystalline cellulose;
preferably, the lubricant is selected from one or more of magnesium stearate, stearic acid, sodium stearyl fumarate, glyceryl behenate, hydrogenated castor oil and sodium lauryl sulfate; and preferably magnesium stearate or stearic acid.
Preferably, in parts by weight, a ratio of the mass of the drug-containing sustained-release layer to the mass of the drug-containing tablet core layer is 12.5-1:1, and preferably 5-1:1;
preferably, a ratio of the mass of the drug-containing immediate-release layer to the mass of the drug-containing tablet core layer is 10-1: 1, and preferably 5-1:1.

Another objective of the present invention is to provide a method for preparing the aforementioned controlled-release tablet, including the following steps:
(1) subjecting a raw material of a drug-containing immediate-release layer to wet granulation to obtain a granule of the drug-containing immediate-release layer;
(2) subjecting a raw material of a drug-containing sustained-release layer to wet granulation to obtain a granule of the drug-containing sustained-release layer;
(3) subjecting a raw material of a plain tablet core to wet granulation, tableting, and then sequentially coating with a first layer of isolation coating, a second layer of enteric coating, and a third layer of isolation coating to obtain a drug-containing tablet core; and
(4) placing the drug-containing tablet core onto the granule of the drug-containing sustained-release layer, pre-compressing, then placing the granule of the drug-containing immediate-release layer onto the pre-compressed tablet, tableting, and coating.

Preferably, the wet granulation process in the step (1) or (2) is to dissolve the adhesive in water or an ethanol solution, then add other raw materials except the lubricant for wet granulation, pass the wet-granulated granules through a 1,000-8,000 µm sieve, dry the granules, pass the dried granules through a 1,000-8,000 µm sieve, add the lubricant, and mix.

Preferably, the pre-compressing pressure in the step (4) is 0.1-2 KN, and preferably 0.1-0.5 KN; and the tableting pressure is 5-25 KN, and preferably 8-20 KN.

Preferably, a method for formulating coating solutions of the first layer of isolation coating and the third layer of isolation coating in the step (3) includes: adding water and stirring to form a vortex, then adding the material of isolation coating film into the water, stirring, and then adding the plasticizer and stirring for more than 45 min; and the method for formulating the coating solution required for the second layer of enteric coating includes: firstly adding the additive into an aqueous solution or a 70%-95% ethanol solution, stirring using a high shear mixer at a rotation speed of 8,000-11,000 for 8-15 min to obtain A; and adding the A into the material of the enteric coating, stirring for 0.3-0.8 h, and then passing through a 50-200 µm sieve.

Preferably, the dissolution of loxoprofen sodium in the controlled-release tablet is 20-55% in 2 h, and 80-100% in 10 h.

A further objective of the present invention is to provide use of the aforementioned loxoprofen sodium controlled-release tablet or a controlled-release tablet prepared by the aforementioned preparation method in preparation of a drug for treating a painful disease.

Compared with the prior art, the present invention has the following beneficial effects:
(1) Currently, there is no enteric-coated preparation of loxoprofen sodium. Studies have shown that loxoprofen sodium is a strong basic and weak acidic salt. When dissolved in water, it produces OH⁻ ions, which will increase the local pH and destroy the enteric coating film, causing the API in the tablet core to be released through the enteric coating film and thus failing to achieve the sustained-release effect of the tablet core. In the present invention the enteric coating film is isolated in a specific isolation coating film, and specific coating order, process, coating materials, coating weight gain, etc. are used to synergistically protect the stability of the enteric coating film, so that the drug is released at a specific site to achieve a sustained release effect.
(2) In the present invention, the effect of sustained release of the loxoprofen sodium tablet core is achieved by designing the tablet structure of the core-coated tablet, the components of each coating film and the structure of the controlled-release tablet, achieving an in vivo effective period of 12 h and improving patient compliance. However, the commercially-available coating film will be damaged due to the pressure exerted by compressing the shell on the coated tablet core after the tablet core is coated and compressed into a core-coated tablet, which will lead to the early release of the tablet core and cannot achieve the effect of sustained release of the tablet core.
(3) The present invention has creatively compounded the raw materials suitable for the enteric coating film and isolation coating film of the present invention through multiple experiments, and combined with a unique tablet design at the same time, the drug release can achieve the expected release effect.

### Explanation of terms

"Coating weight gain" appearing in the context of the present invention refers to a ratio of the weight of the coating material to the weight of the tablet before coating.

### BRIEF DESCRIPTION OF DRAWINGS

FIG. 1 is a dissolution curve of Example 4;
FIG. 2 is a dissolution curve of Example 5;
FIG. 3 is a dissolution curve of Example 6;
FIG. 4 is a dissolution curve of Example 2 and Comparative Example 1;
FIG. 5 is a RSD curve of Example 2 and Comparative Example 2;
FIG. 6 is a dissolution curve of Example 1 and Comparative Example 3;
FIG. 7 is a dissolution curve of Example 3 and Comparative Example 4;
FIG. 8 is a dissolution curve of Example 1 and Comparative Example 5;
FIG. 9 is a dissolution curve of Example 3 and Comparative Example 6;
FIG. 10 is a dissolution curve of Example 1 and Comparative Example 9;
FIG. 11 is a dissolution curve of Example 2 and Comparative Example 10;
FIG. 12 is a dissolution curve of Example 5 and Comparative Example 11.

### DETAILED DESCRIPTION

The present invention will be further described below with reference to specific examples.

Unless otherwise specified in the following examples, all materials are commercially available.

The conditions of dissolution methods used in the following examples and comparative examples are as follows: General Principles of China Pharmacopoeia, 2020 Edition 0931 Determination of Dissolution and Release; the second slurry method at 50 rpm, pH 4.5, and 500 ml.

The dissolution determination method used in the following examples and comparative examples is as follows: after sampling, the absorbance of loxoprofen sodium dihydrate is detected at 222 nm using a high performance liquid chromatograph, and the dissolution of it is calculated.

The following EUDRAGIT L30D-55^{®} is a mixture of methacrylic acid and ethyl acrylate in a mass ratio of 1: 1. EUDRAGIT S100^{®} is a mixture of methacrylic acid and methyl methacrylate in a mass ratio of 1:2.

### Example 1

The formula of the drug-containing tablet core layer of loxoprofen sodium in this example was as shown in Table 1.

**Table 1**

| | Component | Content (%) | Formulation (g) |
|---|---|---|---|
| Isolation coating | HPMC-E5 | 11.43 | 12 |
| | TEC | 1.90 | 2 |
| | Water | 86.67 | 91 |
| | Total | 100.00 | 105 |
| Enteric coating | EUDRAGIT L30D-55^{®} (pH 5.5) | 30.00 | 24 |
| | Talcum powder | 5.00 | 4 |
| | TEC | 2.50 | 2 |
| | Water | 62.50 | 50 |
| | Total | 100.00 | 80 |

| | Component | Content (%) | Unit formulation (mg) |
|---|---|---|---|
| Plain tablet core | Loxoprofen sodium dihydrate | 40 | 24 |
| | Lactose G200 | 36.7 | 22 |
| | Microcrystalline Cellulose PH101 | 18.7 | 11.2 |
| | Crosslinked sodium carboxylmethyl cellulose | 2.7 | 1.6 |
| | Magnesium Stearate | 2.0 | 1.2 |
| | Total | 100.0 | 60 |

The preparation method was as follows:
1) Preparation of granules of a tablet core layer: 24 g of a loxoprofen sodium dihydrate, 1.6 g of crosslinked carboxymethyl cellulose sodium, 22 g of lactose G200, and 11.2 g of microcrystalline cellulose PH101 were premixed to obtain a mixture; the mixture was wet granulated, dried, mixed with 1.2 g of magnesium stearate, and sieved to obtain total mixed granules of tablet core layer for plain tablet cores for later use.
2) Tablet core tableting: the total mixed granules of tablet core layer were used as the raw material for the plain tablet cores, and a circular punch die with a suitable diameter was selected to perform conventional tableting to prepare plain tablets of the tablet core;
3) Tablet core coating: an isolation coating solution (the raw materials of the isolation coating film was added into water, stirred, then added with the plasticizer and stirred for more than 45 min to obtain the isolation coating solution) was formulated, the plain tablet was placed into a coating pot and preheated, and coating was started until the tablet bed temperature reached 45°C, the tablet bed temperature was controlled at 44°C, the tablet core was subjected to the first time of isolation coating, with a target weight gain of 5%; after the isolation coating was completed, an enteric coating solution (firstly the anti-adhesive and the plasticizer were added into an aqueous solution, and stirred with a high shear mixer at a rotation speed of 8,000 for 15 min to obtain A; the A was added into the enteric coating material, stir for 0.5h , and then pass through a 60-80 mesh sieve to obtain), preheat the tablet core coated with the isolation coating until the tablet bed temperature reaches 35°C, start enteric coating, and control the tablet bed temperature at 30°C. After reaching the target weight gain of 16%, a second isolation film coating is performed. The coating process is the same as the first isolation coating. After the coating is completed, the tablet core is prepared for use.

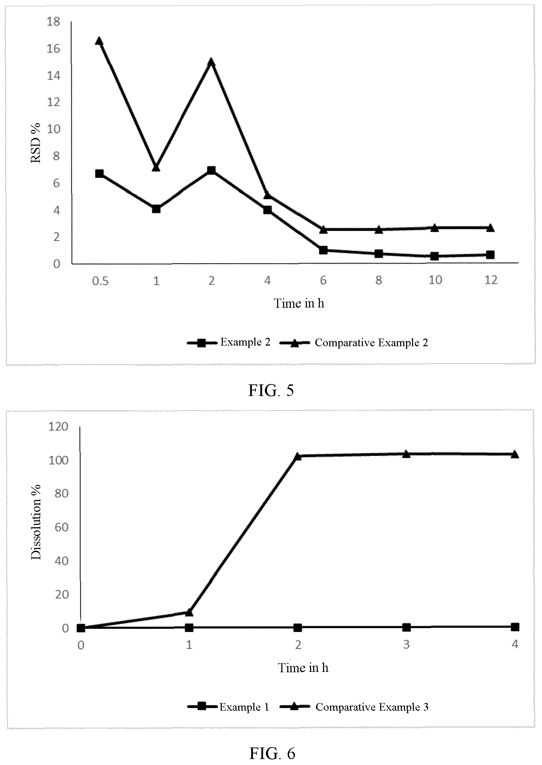

The dissolution curves of this example were shown in FIGs. 6, 8 and 10.

### Example 2

The formula of the drug-containing tablet core layer of loxoprofen sodium in this example was as shown in Table 2.

**Table 2**

| | Component | Content (%) | Formulation (g) |
|---|---|---|---|
| Isolation coating | HPMC-E5 | 8.98 | 25.03 |
| | TEC | 1.35 | 3.75 |
| | Water | 86.68 | 250 |
| | Total | 100.00 | 278.78 |
| Enteric coating | EUDRAGIT L30D-55^{®} (pH 5.5) | 29.17 | 83.38 |
| | Talcum powder | 4.37 | 12.5 |
| | TEC | 2.25 | 6.45 |
| | Water | 64.20 | 183.47 |
| | Total | 100.00 | 285.8 |

| | Component | Content (%) | Unit formulation (mg) |
|---|---|---|---|
| Plain tablet core | Loxoprofen sodium dihydrate | 40 | 24 |
| | Lactose G200 | 36.7 | 22 |
| | Microcrystalline Cellulose PH101 | 18.7 | 11.2 |
| | Crosslinked sodium carboxylmethyl cellulose | 2.7 | 1.6 |
| | Magnesium Stearate | 2.0 | 1.2 |
| | Total | 100.0 | 60 |

The preparation method was as follows:
1)Preparation of granules of tablet core layer: the preparation method was the same as that in Example 1.
2)Tablet core tableting: the total mixed granules of tablet core layer were used as the raw material for the plain tablet cores, and a circular punch die with a suitable diameter was selected to perform conventional tableting to prepare plain tablets of the tablet core;
3) tablet core coating: Prepare isolation coating solution (same as Example 1), place the plain tablets in the coating pot for preheating until the tablet bed temperature reaches 43°C, start coating, control the tablet bed temperature at 43°C, and perform the first isolation coating on the tablet core, with a target weight gain of 2%; after the isolation coating is completed, prepare enteric coating solution (same as Example 1), preheat the tablet core coated with the isolation coating until the tablet bed temperature reaches 33°C, start enteric coating, control the tablet bed temperature at 28°C, and after reaching the target weight gain of 15%, perform the second isolation film coating. The coating process is the same as the first isolation coating. After the coating is completed, the tablet core is prepared for use.

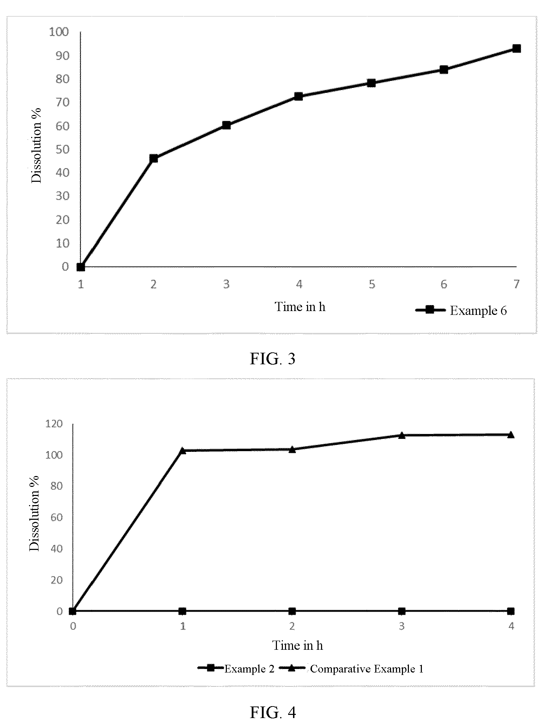

The dissolution curves of this example were shown in Figs. 4, 5 and 11.

### Example 3

The formula of the drug-containing tablet core layer of loxoprofen sodium in this example was as shown in Table 3.

**Table 3**

| | Component | Content (%) | Formulation (g) |
|---|---|---|---|
| Isolation coating | HPMC-E5 | 11.43 | 12 |
| | TEC | 1.90 | 2 |
| | Water | 86.67 | 91 |
| | Total | 100.00 | 105 |
| Enteric coating | EUDRAGIT S100^{®} (pH 7.0) | 5.60 | 14 |
| | Talcum powder | 1.60 | 4 |
| | TEC | 2.16 | 5.4 |
| | Water + ethanol | 4.92+85.72 | 12.3+214.3 |
| | Total | 100.00 | 250 |

| | Component | Content (%) | Unit formulation (mg) |
|---|---|---|---|
| Plain tablet core | Loxoprofen sodium dihydrate | 40 | 24 |
| | Lactose G200 | 36.7 | 22 |
| | Pregelatinized starch | 18.7 | 11.2 |
| | Cross-linked sodium carboxymethyl starch | 2.7 | 1.6 |
| | Magnesium Stearate | 2.0 | 1.2 |
| | Total | 100.0 | 60 |

The preparation method was as follows:
1)Preparation of granules of tablet core layer: the preparation method was the same as that in Example 1.
2) Tablet core tableting: the total mixed granules of tablet core layer were used as the raw material for the plain tablet cores, and a circular punch die with a suitable diameter was selected to perform conventional tableting to prepare plain tablets of the tablet core;
3) Tablet core coating: an isolation coating solution (same as those in Example 1) was formulated, the plain tablet into a coating pot for preheating until the tablet bed temperature reaches 47°C, start coating, control the tablet bed temperature at 45°C, and perform the first barrier coating on the tablet core, with a target weight gain of 10%; after the barrier coating is completed, prepare an enteric coating solution (same as in Example 1), preheat the tablet core coated with the barrier coating until the tablet bed temperature reaches 37°C, start enteric coating, control the tablet bed temperature at 33°C, and after reaching the target weight gain of 25%, perform a second barrier film coating, and the coating process is the same as the first barrier coating. After the coating is completed, the tablet core is prepared for use.

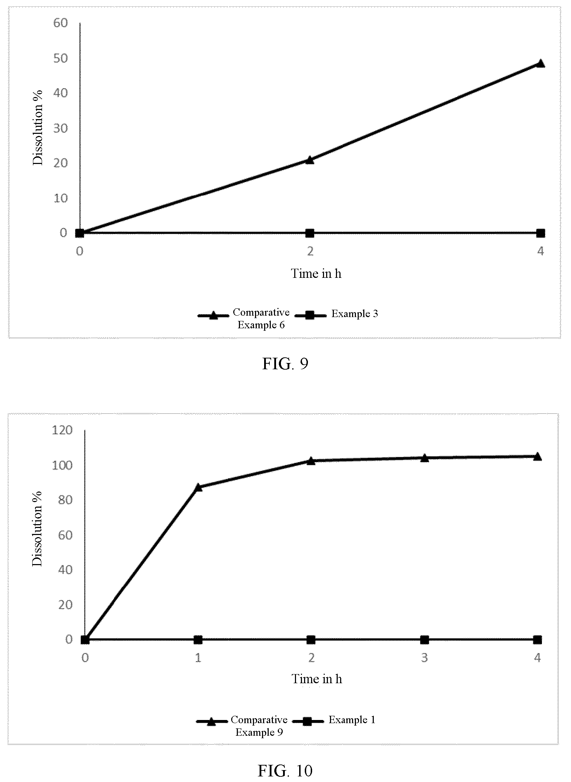

The dissolution curves of this example were shown in FIG. 9.

### Example 4

The formula of the controlled-release tablet of loxoprofen sodium of this example was shown in Table 4 below.

**Table 4**

| Tablet layer | Material Name | Content (%) | Unit formulation (mg) |
|---|---|---|---|
| Immediate-release layer | Loxoprofen sodium dihydrate | 12 | 24 |
| | Lactose G200 | 40.5 | 81 |
| | Microcrystalline Cellulose PH101 | 34 | 68 |
| | Crosslinked sodium carboxylmethyl cellulose | 5.5 | 11 |
| | Magnesium Stearate | 1.5 | 3 |
| | PVP-30 | 6.5 | 13 |
| | Total | 100 | 200 |
| Tablet core layer | Drug-containing tablet core layer prepared in Example 1 | | |
| Sustained-release layer | Loxoprofen sodium dihydrate | 22.5 | 55 |
| | HPMC-K4M | 15 | 30 |
| | HPMC-K100M | 15 | 30 |
| | Lactose G200 | 34 | 68 |
| | PVP-K30 (internal addition) | 4 | 8 |
| | PVP-K30 (external addition) | 3 | 6 |
| | Magnesium Stearate | 1.5 | 3 |
| | Total | 100 | 200 |

The preparation method was as follows:
1)Preparation of granules of immediate-release layer: 24 g of a loxoprofen sodium dihydrate, 11 g of crosslinked sodium carboxymethyl cellulose, 81 g of lactose G200, 68 g of microcrystalline cellulose PH101, and 13 g of PVP-30 were premixed to obtain a mixture; the mixture was wet granulated with purified water, dried, and mixed with 3 g of sieved magnesium stearate to obtain total mixed granules of immediate-release layer for later use.
2)Preparation of granules of sustained-release layer: 55 g of a loxoprofen sodium dihydrate, 30 g of HPMC-K4M, 30 g of HPMC-K100M and 8 g of PVP-K30 were premixed to obtain a mixture; 6 g of PVP-K30 was added to an ethanol solution, the mixture was wet granulated, dried, and mixed with 3 g of sieved magnesium stearate to obtain total mixed granules of sustained-release layer for later use.
3) Tableting: a prescribed amount of the granules of immediate-release layer was placed in a punch die of a tablet press, and the tablet core obtained in Example 1 was placed on the immediate-release layer, and pre-compressed; then a prescribed amount of the granules of sustained-release layer was placed in a punch die of a tablet press, and compressed into tablets under adjusted appropriate main pressure.
4)Film coating: the tablets prepared in step 3) were conventionally film coated with Opadry with a weight gain of 2% to obtain the product.

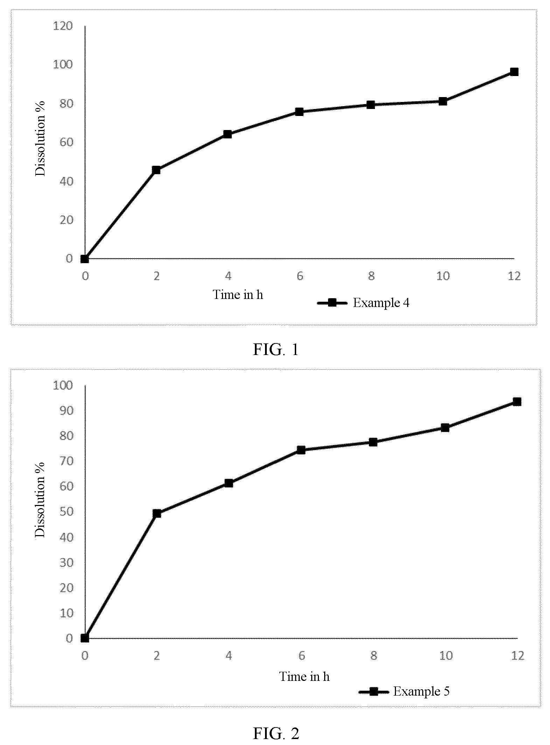

The dissolution curve of the controlled-release tablet prepared in this example was shown in FIG. 1.

### Example 5

The formula of the controlled-release tablet of loxoprofen sodium of this example was shown in Table 5 below.

**Table 5**

| Tablet layer | Material Name | Content (%) | Unit formulation (mg) |
|---|---|---|---|
| Immediate-release layer | Loxoprofen sodium dihydrate | 18 | 36 |
| | Pregelatinized starch | 30 | 60 |
| | Microcrystalline Cellulose PH101 | 35 | 70 |
| | Crosslinked sodium carboxylmethyl cellulose | 6 | 12 |
| | Magnesium Stearate | 3.5 | 7 |
| | Sodium carboxymethyl cellulose | 7.5 | 15 |
| | Total | 100 | 200 |
| Tablet core layer | Same as the tablet core layer prepared in Example 2 | | |
| Sustained-release layer | Loxoprofen sodium dihydrate | 30 | 60 |
| | HPMC-K15M | 12.5 | 25 |
| | Sodium alginate | 14 | 28 |
| | Microcrystalline cellulose | 34 | 68 |
| | Sodium carboxymethyl cellulose (internal addition) | 8 | 16 |
| | Magnesium Stearate | 1.5 | 3 |
| | Total | 100 | 100 |

The preparation method was as follows:
1)Preparation of immediate-release layer granules: premix 36 g of loxoprofen sodium dihydrate, 12 g of crosslinked sodium carboxymethyl cellulose, 60 g of pregelatinized starch, and 70 g of microcrystalline cellulose PH101 to obtain a mixture; add 15 g of sodium carboxymethyl cellulose to purified water to prepare a solution, wet granulate the mixture, dry it, and mix it with 7 g of magnesium stearate to obtain immediate-release layer mixed granules for later use.
2)Preparation of granules of sustained-release layer: 60 g of a loxoprofen sodium dihydrate, 25 g of HPMC-K15M, 28 g of sodium alginate, 68 g of microcrystalline cellulose and 16 g of sodium carboxymethyl cellulose were premixed to obtain a mixture; and the mixture was wet granulated, dried and mixed with 3 g of magnesium stearate to obtain total mixed granules of sustained-release layer for later use.
3) Tableting: a prescribed amount of the granules of immediate-release layer was placed in a punch die of a tablet press, and the tablet core obtained in Example 2 was placed on the immediate-release layer, and pre-compressed; then a prescribed amount of the granules of sustained-release layer was placed in a punch die of a tablet press, and compressed into tablets under adjusted appropriate main pressure.
4)Film coating: the tablets prepared in step 3) were conventionally film coated with Opadry with a weight gain of 2% to obtain the product.

The dissolution curves of this example were shown in FIG. 2.

### Example 6

The formula of the controlled-release tablet of loxoprofen sodium of this example was shown in Table 6 below.

**Table 6**

| Tablet layer | Material Name | Formulation (%) | Unit formulation (mg) |
|---|---|---|---|
| Immediate-release layer | Loxoprofen sodium dihydrate | 13 | 26 |
| | Lactose G200 | 36 | 72 |
| | Microcrystalline Cellulose PH101 | 35 | 70 |
| | Crosslinked sodium carboxylmethyl cellulose | 4 | 8 |
| | Magnesium Stearate | 4 | 8 |
| | Hydroxypropyl methylcellulose | 10 | 20 |
| | Total | 100 | 200 |
| Tablet core layer | Same as the tablet core layer prepared in Example 3 | | |
| Sustained-release layer | Loxoprofen sodium dihydrate | 35 | 70 |
| | HPMC-E30LV | 10 | 20 |
| | HPMC-K100LV | 18 | 36 |
| | Lactose G200 | 30 | 60 |
| | Hydroxypropyl methylcellulose (internal addition) | 4 | 8 |
| | Magnesium Stearate | 3 | 6 |
| | Total | 100 | 200 |

### Preparation method:

1) Preparation of granules of immediate-release layer: 26 g of a loxoprofen sodium dihydrate, 8 g of crosslinked carboxymethyl cellulose sodium, 72 g of lactose G200, 70 g of microcrystalline cellulose PH101, and 20 g of hydroxypropyl methylcellulose were premixed to obtain a mixture; and the mixture was wet granulated with purified water, dried, and mixed with 8 g of sieved magnesium stearate to obtain total mixed granules of immediate-release layer for later use.
2) Preparation of granules of sustained-release layer: 70 g of a loxoprofen sodium dihydrate, 20 g of HPMC-E30LV, 36 g of HPMC-K100LV, 60 g of lactose G200 and 8 g of hydroxypropyl methylcellulose were premixed to obtain a mixture; and the mixture was wet granulated, dried, and mixed with 6 g of sieved magnesium stearate to obtain total mixed granules of sustained-release layer for later use.
3) Tableting: a prescribed amount of the granules of immediate-release layer was placed in a punch die of a tablet press, and the tablet core obtained in Example 3 was placed on the immediate-release layer, and pre-compressed; then a prescribed amount of the granules of sustained-release layer was placed in a punch die of a tablet press, and compressed into tablets under adjusted appropriate main pressure.
4) Film coating: the tablets prepared in step 3) were conventionally film coated with Opadry with a weight gain of 2% to obtain the product. The dissolution curves of this example were shown in FIG. 3.

### Comparative Example 1

The difference between this comparative example and Example 2 was that the coating of the tablet core was different. This comparative example did not carry out isolation coating, but only carries out enteric coating of the tablet core, and the rest was the same as those in Example 2. The dissolution curves of Comparative Example 1 and Example 2 were shown in FIG. 4.

Result analysis: the table showed the dissolution results of the tablet cores, and it required that the tablet core was intact or the dissolution rate of the tablet core was less than 10% within 0-4 h. Within 0-4 h, in a medium of pH 4.5, the tablet core of Example 2 was intact and qualified; while the tablet core of Comparative Example 1 released 102.6% at 1 h, indicating that the tablet core was completely released and it was failed.

### Comparative Example 2

The difference between this comparative example and Example 2 was that the isolation coating was not carried out for the second time in the process for the drug-containing tablet core layer, and the rest was the same as those in Example 2.

The RSDs of Comparative Example 2 and Example 2 were shown in FIG. 5.

Result analysis: the RSDs of Example 2 were each less than 10%, which was relatively stable and qualified; while the RSD of Comparative Example 2 reached a maximum of 16.6% and a minimum of 2.6%, with large variation and unqualified consistency.

### Comparative Example 3

The difference between this comparative example and Example 1 was that the weight gain of enteric coating was 7.03%, and the rest was the same as those in Example 1.

The dissolution curves of Comparative Example 3 and Example 1 were shown in FIG. 6.

Result analysis: within 0-4 h, in the medium of pH 4.5, the tablet core of Example 1 was intact and qualified; while the tablet core of Comparative Example 3 released 9.3% at 1 h, and the dissolution reached 102.3% at 2 h, indicating that the tablet core was completely released and it was failed.

### Comparative Example 4

The difference between this comparative example and Example 3 was that the weight gain of enteric coating was 4.50%, and the rest was the same as those in Example 3.

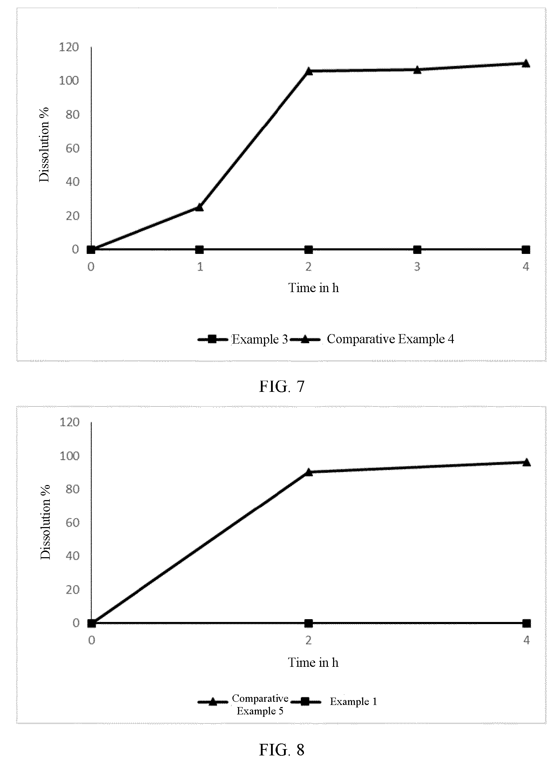

The dissolution curves of Comparative Example 4 and Example 3 were shown in FIG. 7.

Result analysis: within 0-4 h, in the medium of pH 4.5, the tablet core of Example 3 was intact and qualified; while the tablet core of Comparative Example 4 released 25.1% at 1 h, and the dissolution reached 105.9% at 2 h, indicating that the tablet core was completely released.

### Comparative Example 5

The difference between this comparative example and Example 1 was that the isolation coating material was the commercially-available OPADRY^{®} II isolation coating material, which was a mixture of polyvinyl alcohol and polyethylene glycol 3350, and the rest was the same as those in Example 1.

The dissolution curves of Comparative Example 5 and Example 1 were shown in FIG. 8.

Result analysis: the dissolution rate of Comparative Example 5 was 90.6% at 2 h, so that the release was too fast and thus it was failed.

### Comparative Example 6

The difference between this comparative example and Example 3 was that the ratio of the plasticizer and the anti-adhesive agent in the raw material of enteric coating was different, and the rest was the same as those in Example 3. The specific ingredients were shown in Table 7.

**Table 7**

| | Component | Content (%) | Formulation (g) |
|---|---|---|---|
| Isolation coating | HPMC-E5 | 11.43 | 12 |
| | TEC | 1.90 | 2 |
| | Water | 86.67 | 91 |
| | Total | 100.00 | 105 |
| Enteric coating | EUDRAGIT S100^{®} (pH 7.0) | 5.60 | 14 |
| | Talcum powder | 3.2 | 8 |
| | TEC | 0.56 | 1.4 |
| | Water + ethanol | 4.92+85.72 | 12.3+214.3 |
| | Total | 100.00 | 250 |
| Plain tablet core | Same as Example 3 | | |

The dissolution curves of Comparative Example 6 and Example 3 were shown in FIG. 9.

Result analysis of dissolution: the dissolution of Comparative Example 6 was 48.6% at 4 h, so that the release was too fast and thus it was failed.

### Comparative Example 7

The difference between this comparative example and Example 4 was that the adhesive in the drug-containing sustained-release layer was different, specifically HPC-EF, and the rest was the same as those in Example 4.

Example 4 and Comparative Example 7 were examined in terms of appearance and friability. The experimental data was shown in Table 8 below.

**Table 8**

| | Exampl e 4 | Comparative Example 7 |
|---|---|---|
| Friability | 0.19% | 0.27% |
| Appearance | Smooth | Severe edge knocking |

Result analysis: In the case of different types of adhesives in the drug-containing sustained-release layer, Example 4 had a smooth appearance and was qualified; while Comparative Example 7 had chipped edges and was failed.

### Comparative Example 8

The difference between this comparative example and Example 4 was that there was no adhesive in the drug-containing immediate-release layer, specifically as shown in Table 9 below.

**Table 9**

| Tablet layer | Material Name | Formulation (%) | Unit formulation (mg) |
|---|---|---|---|
| Immediate-release layer | Loxoprofen sodium dihydrate | 12 | 24 |
| | Lactose G200 | 46.5 | 93 |
| | Microcrystalline Cellulose PH101 | 34 | 68 |
| | Crosslinked sodium carboxylmethyl cellulose | 5.5 | 11 |
| | Magnesium Stearate | 1.5 | 3 |
| | Total | 100 | 200 |
| Tablet core layer | Same as the tablet core layer prepared in Example 1 | | |
| Sustained-release layer | Loxoprofen sodium dihydrate | 22.5 | 55 |
| | HPMC-K4M | 15 | 30 |
| | HPMC-K100M | 15 | 30 |
| | Lactose G200 | 34 | 68 |
| | PVP-30 (internal addition) | 4 | 8 |
| | PVP-30 (external addition) | 3 | 6 |
| | Magnesium Stearate | 1.5 | 3 |
| | Total | 100 | 200 |

Example 4 and Comparative Example 8 were examined in terms of appearance and friability. The experimental data was shown in Table 10 below.

**Table 10**

| | Example 4 | Comparative Example 8 |
|---|---|---|
| Friability | 0.19% | 91% |
| Appearance | Smooth | / |

Result analysis: in the case of no adhesive in the immediate-release layer, the friability of Example 4 was 0.19%, which was qualified; and the friability of Comparative Example 8 reached 91%, which was failed.

### Comparative Example 9

The difference between this comparative example and Example 1 was that Colorcon 93O was used as the enteric coating material, which was a mixture of methacrylic acid and ethyl acrylate copolymers, and the rest was the same as those in Example 1.

The dissolution curves of Comparative Example 9 and Example 1 were shown in FIG. 10.

Result analysis: within 0-4 h, in the medium of pH 4.5, the tablet core of Comparative Example 9 released 87.2% at 1 h, and the dissolution reached 102.5% at 2 h, indicating that the tablet core was completely released and it was failed.

### Comparative Example 10

The difference between this comparative example and Example 2 was that the coating order was different. Specifically, the plain tablet core was coated with isolation coating layers for two times and then coated with an enteric film, and the specific coating parameters were the same as those in Example 2.

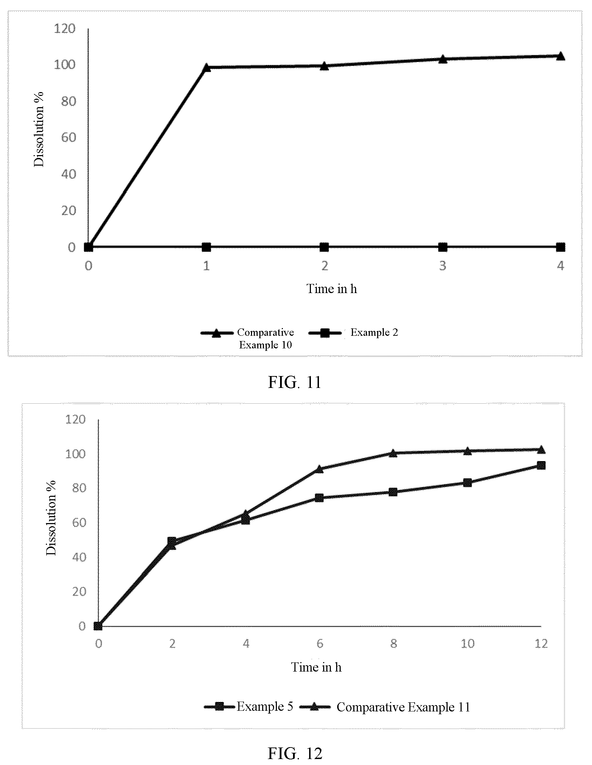

The dissolution curves of Comparative Example 10 and Example 2 were shown in FIG. 11.

Result analysis: within 0-4 h, in the medium of pH 4.5, the tablet core of comparative example 10 released 98.7% at 1 h, and the dissolution reached 103.1% at 3 h, indicating that the tablet core was completely released.

### Comparative Example 11

The difference between this comparative example and Example 5 was that the tablet core layer was inconsistent. The tablet core used in this comparative example was the tablet core prepared in Comparative Example 2, and the rest was consistent with those of Example 5.

The dissolution curves of Comparative Example 11 and Example 5 were shown in FIG. 12.

Result analysis: in the case of inconsistent tablet core layers, the dissolution of Comparative Example 11 at 8 h was 100.6%, indicating that the tablet core was completely released and did not achieve the effect of sustained release for 12 h, and thus it was failed; and the dissolution of Example 5 at 12 h was 93.5%, which achieved the effect of sustained release for 12 h, and thus it was qualified.

The aforementioned description is only preferred embodiments of the present invention, rather than limiting the present invention, and any modification, equivalent substitution and improvement within the spirit and principle of the present invention should be included in the claimed scope of the present invention.

## Claims

1. A loxoprofen sodium controlled-release tablet, comprising a drug-containing immediate-release layer, a drug-containing tablet core layer and a drug-containing sustained-release layer, wherein the drug-containing tablet core layer comprises a plain tablet core, an enteric coating film and an isolation coating film; and the enteric coating film does not contact with the plain tablet core, the drug-containing immediate-release layer and the drug-containing sustained-release layer.

2. The controlled-release tablet according to claim 1, wherein the enteric coating film is in direct contact only with the isolation coating film, and an exterior of the plain tablet core in the drug-containing tablet core layer is, from the inside to the outside, an isolation coating film 1, the enteric coating film and an isolation coating film 2.

3. The controlled-release tablet according to claim 1, wherein a raw material of the isolation coating film comprises an isolation coating material selected from one or more of hydroxypropyl methylcellulose, hydroxypropyl cellulose, ethyl cellulose, and polyvinyl alcohol; the content of the isolation coating material in the isolation coating film is 5-20%, and a coating weight gain of the isolation coating film is 2%-10%.

4. The controlled-release tablet according to claim 1, wherein a raw material of the enteric coating film comprises an enteric coating material selected from one or more of methacrylic acid, acrylate, methacrylate, polymethacrylic acid, polymethacrylate, trimethylaminoethyl methacrylate chloride, and polyvinyl acetate phthalate, the content of the enteric coating material in the enteric coating film is 5-35%, and the coating weight gain of the enteric coating film is 5%-30%.

5. The controlled-release tablet according to claim 1, wherein the enteric coating material is a mixture of methacrylic acid and ethyl acrylate, the coating weight gain is 15-25%; or the enteric coating material is a mixture of methacrylic acid and methyl methacrylate, and the coating weight gain is 6-30%.

6. The controlled-release tablet according to claim 1, wherein a raw material of the enteric coating film further comprises an additive that comprises a plasticizer and an anti-adherent agent in a mass ratio of 0.25-5:1.

7. The controlled-release tablet according to claim 6, wherein the additive comprises a plasticizer and an anti-adherent agent in a mass ratio of 0.25-4:1.

8. The controlled-release tablet according to claim 1, wherein the raw material of the isolation coating film further comprises a plasticizer.

9. The controlled-release tablet according to claim 5, wherein the plasticizer is each selected from one or more of triethyl citrate, polyethylene glycol 6000, tributyl citrate and dibutyl sebacate; and the anti-adherent agent is selected from one or more of talc, magnesium stearate and glyceryl monostearate.

10. The controlled-release tablet according to claim 1, wherein
a raw material of the plain tablet core comprises the following components: loxoprofen sodium, a filler, a disintegrant, and a lubricant;
a raw material of the drug-containing immediate-release layer comprises the following components: loxoprofen sodium, a filler, an adhesive, a disintegrant, and a lubricant; and
a raw material of the drug-containing sustained-release layer comprises the following components: loxoprofen sodium, a sustained-release polymer, a filler, an adhesive, and a lubricant.

11. The controlled-release tablet according to claim 10, wherein the raw material of the plain tablet core comprises the following components: 20-65 parts of loxoprofen sodium, 30-70 parts of the filler, 1-5 parts of the disintegrant, and 1-5 parts of the lubricant;
the raw material of the drug-containing immediate-release layer comprises the following components: 5-25 parts of loxoprofen sodium, 50-80 parts of the filler, 4-10 parts of the adhesive, 2-8 parts of the disintegrant, and 0.5-8 parts of the lubricant; and
the raw material of the drug-containing sustained-release layer comprises the following components: 10-35 parts of loxoprofen sodium, 5-60 parts of the sustained-release polymer, 25-40 parts of the filler, 5-15 parts of the adhesive, and 0.5-8 parts of the lubricant.

12. The controlled-release tablet according to claim 10, wherein the sustained-release polymer is selected from one or more of hydroxypropyl methylcellulose and a polysaccharide gum; the hydroxypropyl methylcellulose is selected from one or more of E30LV, E50LV, K100LV, K4M, K15M and K100M; the polysaccharide gum is selected from one or more of sodium alginate , arabic gum, xanthan gum and locust bean gum; the sustained-release polymer accounts for 5-60% or 7.5-40% of the sustained-release layer; and the adhesive is each selected from one or more of sodium carboxymethyl cellulose, povidone and hydroxypropyl methylcellulose, and preferably povidone.

13. The controlled-release tablet according to claim 1, wherein in parts by weight, a ratio of the mass of the drug-containing sustained-release layer to the mass of the drug-containing tablet core layer is 12.5-1:1; and a ratio of the mass of the drug-containing immediate-release layer to the mass of the drug-containing tablet core layer is 10-1:1.

14. A method for preparing the controlled-release tablet according to any one of claims 1-13, comprising the following steps:
(1) subjecting a raw material of a drug-containing immediate-release layer to wet granulation to obtain a granule of the drug-containing immediate-release layer;
(2) subjecting a raw material of a drug-containing sustained-release layer to wet granulation to obtain a granule of the drug-containing sustained-release layer;
(3) subjecting a raw material of a plain tablet core to wet granulation, tableting, and then sequentially coating with a first layer of isolation coating, a second layer of enteric coating, and a third layer of isolation coating to obtain a drug-containing tablet core; and
(4) placing the drug-containing tablet core onto the granule of the drug-containing sustained-release layer, pre-compressing, then placing the granule of the drug-containing immediate-release layer onto the pre-compressed tablet, tableting, and coating.

15. The preparation method according to claim 14, wherein, in the step (4), a pre-compressing pressure is 0.1-2 KN, or 0.1-0.5 KN; and a tableting pressure is 5-25 KN or 8-20 KN.

16. Use of the loxoprofen sodium controlled-release tablet according to any one of claims 1-13 or a controlled-release tablet prepared by the preparation method according to any one of claims 14-15 in preparation of a drug for treating a painful disease.
